# EUROPEAN PATENT APPLICATION

(11) **EP 1 842 560 A1**
(43) Date of publication of application: **10.10.2007**
(21) Application number: 05781912.0
(22) Date of filing: 31.08.2005
(51) Int. Cl.: A61K 49/00, A61K 49/22

(54) **A HIGH-INTENSITY FOCUSED ULTRASOUND ADJUVANT AND THE SCREENING METHOD THEREOF**

(30) Priority: 10.01.2005 CN 200510000345
(71) Applicant: Chongqing Haifu (Hifu) Technology Co., Ltd., Chongqing 401121 (CN)
(72) Inventor: WANG, Zhilong, Yubei District, Chongquing 401121 (CN); WANG, Zhibiao, Yubei District, Chongquing 401121 (CN); LI, Faqi, Yubei District, Chongquing 401121 (CN); XIAO, Yanbing, Yubei District, Chongquing 401121 (CN); XIAO, Ziwen, Yubei District, Chongquing 401121 (CN); LIU, Liping, Yubei District, Chongquing 401121 (CN)
(74) Representative: Kampfenkel, Klaus
(86) International application number: PCT/CN2005/001367
(87) International publication number: WO 2006/072198

(57) **Abstract**

The present invention discloses an enhancement agent for high intensity focused ultrasound (HIFU) treatment, which is administered to a patient before HIFU treatment and can reduce the level of EEF at the target location to be treated with HIFU. EEF is presented by the expression: *EEF* =*^{ηPt}*/*v* (unit: J/mm³), and refers to the HIFU energy needed to effectively treat a tumor per unit volume of the tumor, wherein, η=0.7; P refers to the total acoustic power of HIFU source (unit: W); t refers to the total time of HIFU treatment (unit: s); V refers to the volume of HIFU-induced lesions (unit: mm³). If the amount of EEF at the target location before administration of the enhancement agent is defined as EEF_{(base)} and the amount of EEF at the target location after administration of the enhancement agent is defined as EEF₍ₘₑₐₛᵤᵣₑₘₑₙₜ₎, the ratio between EEF_{(base)} and EEF₍ₘₑₐₛᵤᵣₑₘₑₙₜ₎ is more than 1, preferably more than 2, and more preferably over 4. The use of the enhancement agent for HIFU treatment of the present invention makes it possible to treat deep-seated tumors. In addition, patients with hepatic tumors can be effectively treated without removal of ribs. Accordingly, the present invention discloses methods for increasing acoustic energy deposition at target location during HIFU treatment and screening the enhancement agents for HIFU treatment.

## Description

### FIELD OF THE PRESENT INVENTION

The present invention is related to the fields of medicine and medical treatment, specifically to the field of ultrasound treatment, and more particularly to an enhancement agent for HIFU treatment, which can increase acoustic energy deposition at the target location during HIFU treatment, and a method for screening the enhancement agents for HIFU treatment.

### BACKGROUND OF THE PRESENT INVENTION

High-intensity focused ultrasound (HIFU) as a new technique to treat tumors and other diseases has already been recognized in clinical applications. HIFU employs focused ultrasound, which provides continuous, high-intensity ultrasound energy at the focus, resulting in instantaneous thermal effects (65-100 °C), cavitation effects, mechanical effects and sonochemical effects, to selectively cause coagulative necrosis at the focus, and prevent tumors from proliferation, invasion and metastasis.

It was demonstrated that the acoustic energy was attenuated exponentially as the transmission distance increased during the ultrasound transmission within the body (Baoqin Liu et al., Chinese Journal of Ultrasound in Medicine, 2002, 18(8):565-568). In addition, the energy during ultrasound transmission in soft tissues was attenuated due to tissue absorption, scattering, refraction, diffraction and the like, among which tissue absorption and scattering are mainly responsible for the energy loss (Ruo Feng and Zhibiao Wang as editors in chief, Practical Ultrasound Therapeutics, Science and Technology Reference Publisher of China, Beijing, 2002.14). Therefore, when the HIFU treatment is used to treat deep-seated and large-sized tumors, the acoustic energy transmitted to the target would be relatively low. Thus, therapeutic efficiency would decrease and the treatment time would be prolonged due to the acoustic energy attenuation.

Of course, although the transmitting power of the therapeutic transducer might be increased in order to improve the therapeutic efficiency, the normal tissue along the pathway of the ultrasound transmission is more likely to be burned.

In addition, at present, when the HIFU technique is clinically applied to a hepatic tumor that is blocked by the ribs in the pathway of the ultrasound transmission, the ribs are usually removed in order to increase the energy deposition at the target location, shorten the treatment time and improve therapeutic effects. Thus the noninvasiveness of HIFU treatment cannot be ensured, which is undesirable for the patients and doctors.

The above problems have disadvantageously limited the use of the HIFU treatment as a technique for clinical practice. Therefore, the technical problems with respect to increasing the energy deposition at target location, effectively treating the deep-seated tumors without damaging the surrounding normal tissue in the acoustic pathway, and treating a hepatic tumor that is blocked by the ribs without removal of the ribs, need to be solved urgently.

### SUMMARY OF THE PRESENT INVENTION

One objective of the present invention is to provide an enhancement agent for high intensity focused ultrasound (HIFU) treatment, which can enhance the acoustic energy deposition at target location during HIFU treatment.

Another objective of the present invention is to provide a method for screening the enhancement agent for HIFU treatment.

A further objective of the present invention is to provide use of an enhancement agent for HIFU treatment to increase the effectiveness of HIFU treatment.

In order to achieve the above objectives, in one embodiment, the present invention provides an enhancement agent for HIFU treatment, wherein, the enhancement agent is a substance that can enhance acoustic energy absorption at the target location to be treated with HIFU after its administration to a biological body, i.e. a substance that can be used to reduce the acoustic energy needed to cause lesions of a target tissue (tumor and non-tumor tissue) per unit volume of the tissue during HIFU treatment. In the present invention, the types of the substances used as the enhancement agents for HIFU treatment are not particularly limited, as long as the substances can change the acoustic environment of the target tissue and promote therapeutic acoustic energy absorption and deposition at the target tissue to effectively decrease the energy efficiency factor (EEF) of the target tissue. Therefore, the enhancement agents for HIFU treatment in the present invention can be solid, liquid or gas.

As used herein, the term "lesion" refers to the substantial change in the physiological state of a tumor tissue, generally refers to the coagulative necrosis of a tumor tissue. Energy efficiency factor (EEF) can be used to quantify the acoustic energy needed to cause lesions of a target tissue per unit volume of the tissue. EEF is presented by the expression of *EEF = ^{ηPt}*/*_{V},* (unit: J/mm³), and refers to the acoustic energy needed to cause lesions of a tumor tissue per unit volume of the tissue, wherein, η refers to the focusing coefficient of a HIFU transducer, which reflects the ultrasound energy focusing capacity of the transducer, here η=0.7; P refers to the total acoustic power of a HIFU source (unit: W), t refers to the total time of HIFU treatment (unit: s); and V refers to the volume of HIFU-induced lesions (unit: mm³). A substance that greatly decreases the EEF of the target tissue after its administration is more suitable to be used as the enhancement agent for HIFU treatment.

In one preferred embodiment, the enhancement agent for HIFU treatment decreases the EEF of the target tissue after its administration. As a result, the ratio between the EEF of the target tissue measured before the administration of the enhancement agent (i.e. EEF_{(base)}) and the EEF of the target tissue measured after the administration of the enhancement agent (i.e. EEF₍ₘₑₐₛᵤᵣₑₘₑₙₜ₎) is more than 1, preferably more than 2, and more preferably over 4. The upper limit of the ratio is not particularly limited and a higher ratio is preferred.

In one further preferred embodiment, the enhancement agent for HIFU treatment is a biocompatible material with a particle size ranging from 10nm-8µm, which can be administered via intravenous, arterial, or topical injections and can decrease the EEF of the target tissue after its administration. Accordingly, the ratio between the EEF of the target tissue before administration of the enhancement agent (i.e. EEF_{(base)}) and the EEF of the target tissue after administration of the enhancement agent (EEF₍ₘₑₐₛᵤᵣₑₘₑₙₜ₎) is more than 1, preferably more than 2, and more preferably over 4. The upper limit of the ratio is not particularly limited and a higher ratio is preferred.

The enhancement agents for HIFU treatment of the present invention may be encapsulated by a lipid membrane, protein membrane or saccharide membrane, or may be in a naked form without being encapsulated. For example, for tissues that are enriched with reticuloendothelial cells, such as liver, spleen, and bone marrow cells, the enhancement agent for HIFU treatment may be encapsulated by a lipid membrane in order to improve the target specificity of the enhancement agent. The enhancement agent for HIFU treatment can be administered in a naked form without being encapsulated in the lipid membrane, protein membrane or saccharide membrane, as long as no blood vessel blockage would be induced when the enhancement agent for HIFU treatment is administered intravenously. Additionally, in order to make the enhancement agent for HIFU treatment according to the present invention target a specific tumor tissue, for example, hepatic tumor, kidney tumor, bone tumor, breast cancer and uterine fibroids, substances having a specific affinity to the tumor tissue or the focus, such as a tumor-specific antibody, may be added to the enhancement agent for HIFU treatment.

In one preferred embodiment of the present invention, the enhancement agent for HIFU treatment comprises a discontinuous phase comprised of a core encapsulated by a membrane-forming material, and a continuous phase comprised of aqueous medium. The discontinuous phase is uniformly dispersed in the continuous phase, and the particle size of the discontinuous phase ranges from 10nm to 8µm; the membrane-forming material is biocompatible and the core consists of gas, liquid or nanometer-sized biocompatible solid. Such enhancement agent for HIFU treatment is suitable to be administered intravenously. For the purpose of clarity and convenience, the enhancement agent for HIFU treatment that consists of a biocompatible gas encapsulated by a membrane-forming material, is hereinafter referred to as a "microbubble" enhancement agent; the enhancement agent for HIFU treatment that consists of the liquid encapsulated by a membrane-forming material, is hereinafter referred to as a "particle" enhancement agent, wherein the liquid is classified into two categories: liquid which does not undergo a liquid-gas phase transition at 38-100°C, and liquid which undergoes a liquid-gas phase transition at 38-100°C (specifically, the liquid will turn into gas within an animal body or human body during HIFU treatment); the enhancement agent for HIFU treatment that consists of nanometer-sized biocompatible solid encapsulated by a membrane-forming material, is hereinafter referred to as a "plasmid" enhancement agent.

In the above embodiment, the amount of the membrane-forming material contained in the enhancement agent is 0.1-100g/L, preferably 0.5-50g/L, and more preferably 0.5-20g/L. The membrane-forming material includes: lipids, such as 3-sn-phosphatidylcholine, 1,2-dipalmitoyl-sn-glycero-3-phosphatidylglycerol sodium salt, 1,2-distearoyl-sn-glycero-3-phosphatidylcholine, sodium 1,2-dipalmitoyl-sn-glycero-3-phosphatidate, 1,2-dipalmitoyl-sn-glycero-3-phosphatidylcholine, phosphatidylserine and hydrogenated phosphatidylserine, cholesterol, and glycolipide; saccharides, including, for example, glucose, fructose, sucrose, starch and the degradation products thereof; proteins, such as albumin, globulin, fibrinogen, fibrin, hemoglobin, and the degradation products of plant proteins and the like.

When using a microbubble enhancement agent, the amount of the gas contained in the enhancement agent is 5-200ml/L, preferably 20-150ml/L, and more preferably 20-100ml/L. The gas includes, for example, air, nitrogen, carbon dioxide, fluorohydrocarbon gas, such as perfluoroethane, perfluoropropane, perfluorobutane, alkane gases, such as butane, cyclobutane, pentane, hexane, sulfur hexafluoride, and the like.

The microbubble ultrasound contrast agents widely used in ultrasound imaging may be used as the enhancement agent for HIFU treatment of the present invention. Thus, the present invention provides use of microbubble ultrasound contrast agents as the enhancement agent for HIFU treatment of the present invention.

When using a particle enhancement agent, if liquid that does not undergo a liquid-gas phase transition at 38-100°C, for example, water, saturated fatty acid, and unsaturated fatty acids, such as soybean oil, peanut oil, and iodized oil, is used, the amount of the liquid contained in the enhancement agent is 5-200g/L, preferably 10-100g/L, and more preferably 20-80g/L; if liquid that undergoes a liquid-gas phase transition at 38-100°C, for example, C₅-C₆ alkanes such as n-pentane, i-pentane and the like., and C₅-C₁₂ fluorohydrocarbons such as perfluoropentane, dihydrodecafluoropentane and the like, is used, the amount of the liquid contained in the enhancement agent is 5-200ml/L, preferably 10-100ml/L, and more preferably 20-80ml/L.

For example, a fat emulsion for injection is an aqueous emulsion of fat, consists of refined soybean oil encapsulated by a phospholipin membrane, which is dispersed in water, and is suitable for intravenous injection. Presently this kind of emulsion is commercially available, including, but not limited to, Intralipos® (fat emulsion injection), OMNILIPID® (fat emulsion injection), and "fat emulsion (long chain)" or "fat emulsion (medium chain triglycerides/long chain triglycerides)" listed in the catalogue of basic medicines of the state (China). These fat emulsions can be used as the enhancement agents for HIFU treatment of the present invention. Thus, the present invention provides use of fat emulsions as the enhancement agents for HIFU treatment of the present invention.

When using a plasmid enhancement agent, the nanometer-sized biocompatible solid includes nanometer-sized magnetic biomaterials such as superparamagnetic iron oxide (SPIO), nanometer-sized hydroxylapatite (HAP), nanometer-sized calcium carbonate and the like. Typically, the nanometer-sized biocompatible solid has a particle size ranging from 1nm to 500nm, preferably from 1nm to 200nm, and more preferably from 10nm to 100nm.

Furthermore, the nanometer-sized biocompatible solids as mentioned above can be used as the enhancement agents of the present invention by themselves. Therefore, the present invention provides use of nanometer-sized biocompatible solids as the enhancement agent of the present invention.

In the embodiment as mentioned above, the enhancement agent may contain an emulsifier. The emulsifier is typically selected from the group consisting of ethylene glycol mono-C₁₆₋₁₈-fatty acid esters, diethylene glycol mono-C₁₆₋₁₈-fatty acid esters, diethylene glycol di-C₁₆₋₁₈-fatty acid esters, triethylene glycol mono-C₁₆₋₁₈-fatty acid esters, sorbitan fatty acid ester (Span type) emulsifiers, polysorbate (Tween type) emulsifiers, polyethylene glycol monolaurate-based emulsifiers, polyoxyethylene laurate-based emulsifiers, 3-sn-phosphatidylcholine (lecithin), cholic acid and the like. The amount of the emulsifier in the enhancement agent is 5-150g/L. In addition, the enhancement agent may also contain a stabilizing agent, such as carboxymethylcellulose sodium (CMC-Na), glycerin and the like. The amount of the carboxymethylcellulose sodium contained in the enhancement agent may be 0.01-10g/L, preferably 0.05-0.6g/L, and more preferably 0.1-0.3g/L. The amount of the glycerin contained in the enhancement agent may be 5-100g/L.

In a more preferred embodiment, an inorganic or organic acid or base may be used to adjust the pH value of the enhancement agent in order to increase the stability of the enhancement agent. When using a particle enhancement agent, if the liquid undergoes a liquid-gas phase transition at 38-100°C, the particle enhancement agent may be adjusted to pH 7.0-9.0, preferably 7.5-8.5. When using a plasmid enhancement agent, the plasmid enhancement agent may be adjusted to pH 3.0-6.5, preferably 5.0-6.0.

The methods for the preparation of the enhancement agent for HIFU treatment of the present invention, specifically, a core encapsulated by a membrane-forming material, are not particularly limited. Generally, the membrane-forming material, the gas, liquid or solid to be encapsulated, the emulsifier, the stabilizing agent and the like are sufficiently mixed and emulsified. For example, the particle fat emulsion can be prepared according to the disclosures of Chinese Patent Application No. 97182319.7 (Entitled: Fat emulsion containing reducing sugar and method of sterilization) or Chinese Patent Application No. 02112860.X (Entitled: Fat emulsion for injection and method for producing the same). The microbubble fluoro-carbon emulsion can be prepared according to the disclosures of Chinese Patent Application No. 96106566.4 (Entitled: Dextrose anhydride albumin ultrasound contrast agent containing perfluorohydrocarbons and method for producing the same), Chinese Patent Application No. 98119011.1 (Entitled: A ultrasound contrast agent for ultrasound diagnosis and method for producing the same) or Chinese Patent Application No. ZL 89100726.1 (Entitled: Method for the preparation of the particle used for ultrasound contrast and the ultrasound contrast agent).

The membrane-forming material of the enhancement agent for HIFU treatment according to the present invention is preferably a biocompatible and degradable biomaterial, such as lipid, such that the enhancement agent can be injected intravenously, transported through the blood circulation smoothly, and then phagocytosed quickly by the tissues of the human body, which are full of reticuloendothelial cells. Therefore, a mass of enhancement agent can be deposited in the tissues of the human body in a certain time, thereby, the ultrasound absorption capacity of the tissue can be significantly enhanced, and the acoustic energy deposition at the target tissue during HIFU treatment can be increased and eventually the effectiveness of clinical HIFU treatment to ablate tumor cells can be improved greatly.

The present invention is further directed to a method for increasing the energy deposition at the target location during the HIFU treatment,
wherein, the method comprises administering an effective dosage of the enhancement agent of the present invention intravenously via continuous and rapid IV instillation or bolus injection to a patient at 0-168h before applying HIFU treatment to a patient. The effective dosage mentioned above varies with the type of tumor, weight of patient, location of tumor, volume of tumor and the like. However, a doctor or a pharmacist can easily determine the suitable dosage for different patients. For example, when using a microbubble enhancement agent, the dosage can be selected from the range of 0.005-0.1ml/kg, preferably 0.01-0.05ml/kg. When using a particle enhancement agent, if liquid that does not undergo a liquid-gas phase transition at 38-100°C is used, the dosage can be selected from the range of 0.01-5ml/kg, preferably 0.01-2.5ml/kg; and if liquid that undergoes a liquid-gas phase transition at 38-100°C is used, the dosage can be selected from the range of 0.005-0.1ml/kg, preferably 0.01-0.05ml/kg. When using a plasmid enhancement agent, the dosage can be selected from the range of 0.1-10ml/kg, preferably 0.1-5ml/kg.

The present invention is also directed to a method for screening the enhancement agent for HIFU treatment, the method comprising:
(a) measuring the Energy Effect Factor (EEF) of a biological tissue to obtain EEF_{(base);}
(b) administering a candidate enhancement agent to the biological tissue;
(c) measuring the Energy Effect Factor (EEF) of the tissue after administration of the candidate enhancement agent to obtain EEF₍ₘₑₐₛᵤᵣₑₘₑₙₜ₎;
(d) comparing the EEF₍ₘₑₐₛᵤᵣₑₘₑₙₜ₎ with the EEF_{(base)} of the tissue and selecting the candidate enhancement agent having a ratio between EEF₍ₘₑₐₛᵤᵣₑₘₑₙₜ₎ to EEF_{(base)} of more than 1, preferably more than 2, and more preferably more than 4.

In addition, the present invention provides a treatment method for diseases, comprising administering the enhancement agent for HIFU treatment to a patient before applying the HIFU treatment to improve the therapeutic acoustic energy absorption capacity of the target tissue to be treated with HIFU.

### DETAILED DESCRIPTION OF THE PRESENT INVENTION

The preparation and some physicochemical parameters of the enhancement agent of the present invention will be described further with reference to the following Examples, and the technical effects of the exemplary enhancement agent of the present invention will be described further with reference to the following animal tests. It should be understood that these Examples and tests are provided for the purpose of illustration only and do not intend to limit the scope of the present invention.

### Example I Preparation of a particle enhancement agent for HIFU treatment

### Example 1-1 Using liquid that does not undergo a liquid-gas phase transition at 38-100 °C for encapsulation.

### Example I-1-1

The following materials were mixed: 4g iodized oil for injection (purchased from Shanghai Chemical Reagent Company), 0.6g yolk lecithin for injection (purchased from Shanghai Chemical Reagent Company) and 1.25g glycerin for injection (purchased from Shanghai Chemical Reagent Company), and this mixture was dissolved and formed an oil phase after heating to 70°C. Distilled water containing 1% (w/v) F-68 emulsifier (purchased from Sigma Company) was added to the oil phase to a final volume of 17.5ml. The mixture was agitated to obtain a coarse emulsion. The coarse emulsion, which was poured into a boiling tube, was emulsified by sonication at a power of 350W for 2 minutes. The resulting uniformly emulsified iodized oil was sterilized through flowing steam at 100°C for 30 minutes. The final product had a pH of 7.5-8.5, iodine content of 0.13g/ml, particle size of less than 1µm and osmotic pressure of 350mosm/kg H₂O.

### Examples I-1-2 to I-1-4

Examples I-1-2 to I-1-4 were prepared according to the same method and procedures described in Example I-1-1 except that the iodized oil for injection was replaced with the soybean oil for injection as the core material, and the yolk lecithin for injection was replaced with the lecithin as the membrane-forming material. The particle enhancement agents for HIFU treatment of the present invention were obtained according to the formulation set forth below in Table 1. The enhancement agents were obtained as white emulsion liquids, which can be administered to animals and human beings via intravenous injection. The parameters of the products are also shown in Table 1.

**Table 1**

| | Example I-1-2 | Example I-1-3 | Example I-1-4 |
|---|---|---|---|
| Concentration of Soybean oil for injection in the enhancement agent (w/v) | 10% | 20% | 10% |
| Amount of Soybean oil for injection | 100g | 200g | 100g |
| Amount of Lecithin for injection | 12g | 12g | 12g |
| Amount of Glycerin for injection | 22g | 22g | 16.7g |
| Final volume after Water for injection added | 1000ml | 1000ml | 1000ml |
| pH (c.a.) | 8 | 8 | 8 |
| Particle size of the discontinuous phase | 0.1-2µm | 1-5µm | 0.5-2µm |
| Osmotic pressure (mosm/kg H₂O) | 300 | 350 | 310 |
| Energy MJ (kcal) | 4.6 (1100) | 8.4 (2000) | 12.6 (3000) |

### Example 1-2 Using liquid that undergoes a liquid-gas phase transition at 38-100°C for encapsulation.

### Example I-2-1

The following materials were mixed to a final volume of 1000ml: 3% (w/v) emulsifier Pluronic F-68 (purchased from Sigma Company), 0.5% (w/v) yolk lecithin (purchased from Shanghai Chemical Reagent Company), 5% (v/v) perfluoropentane (purchased from Sigma Company), and distilled water. The mixture was incubated on ice, sheared, and dispersed at 10000 rpm for 5 minutes to obtain a coarse emulsion. The coarse emulsion was emulsified in a high-pressure homogenizer at 4°C for two times. The resulting emulsion with a particle size of less than 1µm was obtained by filtering through a 1 µm membrane filter. The final emulsion was divided and put into 15ml vials, and then was radiated by C₀₆₀ at 20KGY for 10 hours. The emulsion had a particle concentration of 10⁹/ml and was refrigerated for storage.

### Example 1-2-2

The following materials were mixed to a final volume of 1000ml: 6% (w/v) emulsifier Pluronic F-68 (purchased from Sigma Company), 1% (w/v) yolk lecithin (purchased from Shanghai Chemical Reagent Company), 10% (v/v) perfluoropentane (purchased from Sigma Company), and physiological saline solution. The mixture was incubated on ice, sheared, and dispersed at 10000 rpm for 5 minutes to obtain a coarse emulsion. The coarse emulsion was emulsified in a high-pressure homogenizer at 4°C for two times. The resulting emulsion with a particle size of less than 1µm was obtained by filtering through a 1µm membrane filter. The final emulsion was divided and put into 15ml vials, and then was radiated by Co₆₀ at 20KGY for 10 hours. The emulsion had a particle concentration of 10⁹/ml and was refrigerated for storage.

### Example 1-2-3 to 1-2-6

Fluoro-carbon emulsion enhancement agents for HIFU treatment of the present invention were prepared according to the same method and procedures described in Example I-2-1 with the materials and the amounts thereof set forth in Table 2. The parameters of the products are shown in Table 2.

**Table 2**

| | Example I-2-3 | Example I-2-4 | Example I-2-5 | Example I-2-6 |
|---|---|---|---|---|
| Core material | 2% (v/v) Perfluoropentane | 5% (v/v) Perfluorohexane | 10% (v/v) Perfluorohexane | 10% (v/v) Dihydrodecafluoropentane |
| Lecithin | 1% (w/v) | 2% (w/v) | 2% (w/v) | 2% (w/v) |
| Glycerin | 1% (w/v) | 1 %(w/v) | 1% (w/v) | 1% (w/v) |
| Pluronic F-68 | 5% (w/v) | 3% (w/v) | 5% (w/v) | 5% (w/v) |
| Final volume after distilled water added | 1000ml | 1000ml | 1000ml | 1000ml |
| PH (c.a.) | 6.98 | 7.01 | 6.99 | 7.00 |
| Particle size of the discontinuous phase | 0.5-2µm | 0.5-2µm | 0.1-2µm | 1-2µm |

### Example II Preparation of a plasmid enhancement agent for HIFU treatment

### Example II-1

The following materials were mixed: 2.5g HAP with a particle size ranging from 1nm to 100nm (purchased from the Engineering Research Center for Biomaterials of Sichuan University), 0.3g yolk lecithin for injection (purchased from Shanghai Chemical Reagent Company) and 0.3g CMC-Na (purchased from Shanghai Chemical Reagent Company), and distilled water to a final volume of 100ml. After being uniformly mixed, the mixture was pH-adjusted with acetic acid to pH 5.0. The mixture was sonicated for 2 minutes at a power of 400W with the transmitter of the sonicator positioned 1.5 cm below the surface of the mixture. After sonication, a milk-white, uniformly dispersed, stable suspension was obtained. The particle size of the discontinuous phase of the resulting enhancement agent ranged from 10nm to 1000nm, mainly ranged from 100nm to 500nm.

### Example II-2

The following materials were mixed: 2.5g HAP with a particle size ranging from 1 nm to 100nm (purchased from the Engineering Research Center for Biomaterials of Sichuan University), 0.3g yolk lecithin for injection (purchased from Shanghai Chemical Reagent Company) and 1ml glycerin for injection, and distilled water to a final volume of 100ml. After being uniformly mixed, the mixture was pH-adjusted with acetic acid to pH 5.0. The mixture was sonicated for 2 minutes at a power of 400W with the transmitter of the sonicator positioned 1.5 cm below the surface of the mixture. After sonication, a milk-white, uniformly dispersed, stable suspension was obtained. The particle size of the discontinuous phase of the resulting enhancement agent ranged from 10nm to 1000nm, mainly ranged from 100nm to 500nm.

### Examples II-3 to II-5

Plasmid enhancement agents for HIFU treatment of the present invention were prepared according to the same method and procedures described in Example II-1 with the materials and the amounts thereof set forth in Table 3. The parameters of the products were are shown in Table 3.

**Table 3**

| | Example II-3 | Example II-4 | Example II-5 |
|---|---|---|---|
| Nnanometer-sized HAP (particle size) | 25g/L (1-500nm) | 25g/L (1-500nm) | 50g/L (1-500nm) |
| Lecithin | 0.3g | 0.3g | 0.6g |
| CMC-Na | 0.3g | 0.6g | 0.3g |
| Glycerin for injection | 1ml | 1ml | 2ml |
| Final volume after distilled water added | 100ml | 100ml | 100ml |
| PH (c.a.) | 5.0 | 5.0 | 5.0 |
| Particle size of the discontinuous phase | 10-1000nm | 10-1000nm | 10-1000nm |
| Osmotic pressure (mosm/ kg. H₂O) | 275 (Isosmotic) | 275 (Isosmotic) | 275 (Isosmotic) |

### Example III

The nanometer-sized hydroxylapatite (HAP) purchased from the Engineering Research Center for Biomaterials of Sichuan University was a white powder with a particle size ranging from 10nm to 200nm with a normal distribution. HAP was dissolved in a 9% physiological saline solution to obtain two milk-white suspensions with concentrations of 25g/L and 50g/L, respectively. Prior to use, the suspensions were sonicated at a power of 600W so as to be uniformly dispersed.

### Animal test 1 Combined use of the particle enhancement agent for HIFU treatment as prepared in Example I-1-3 and HIFU therapeutic devices

Fifty New Zealand white rabbits (about 3 months old) with no limitation on sex, which were provided by the Laboratory Animals Center of Chongqing University of Medical Sciences, were equally divided into Group A and Group B. The rabbits in Group A and Group B weighed 2.22 ± 0.21kg and 2.24 ± 0.19kg (P>0.05), respectively.

The New Zealand white rabbits were anaesthetized through intramuscular injection, fastened to the treatment bed of a High-intensity Focused Ultrasound Tumor Therapeutic System Model-JC manufactured by Chongqing Haifu (HIFU) Technology Co. Ltd., and then treated by using this System. The High-intensity Focused Ultrasound Tumor Therapeutic System Model-JC was composed of an adjustable power generator, a B-mode ultrasound monitoring system, a therapeutic transducer, a mechanical motion control system, a treatment bed and an acoustic coupling device. The therapeutic transducer of the System, with a working frequency of 1MHz, diameter of 150mm, and focal distance of 150mm, using standard circulating degassed water with a gas content of no more than 3ppm, can produce a focal region of 2.3×2.4×26mm and deliver an average acoustic intensity of 5500W/cm².

The rabbit livers were pre-scanned by the B-mode scanner of the HIFU therapeutic system. Two slices with an interval of at least 2cm at an exposure depth of 2.0cm were measured. For each rabbit in Group A, the left side of the rabbit liver (the left/middle lobe) was considered as the control lobe (which was administered with physiological saline solution), and the right side of the rabbit liver (the right lobe) was considered as the experimental lobe (which was administered with the enhancement agent for HIFU treatment as prepared in Example I-1-3, and also called the enhancement agent side). The control lobe and experimental lobe were reversely positioned in Group B. The exposure depth of HIFU treatment (i.e., the distance from the skin surface to the focal point) was also 2.0cm. After the liver slices were chosen, the physiological saline solution was delivered via rabbit ear border vein at 50-60 drops/min. After 20 minutes, the left side of the rabbit liver (Group A) or the right side of the rabbit liver (Group B) was exposed to HIFU under single pulse exposure or multi-pulse exposure (line length: 1 cm, scanning speed: 3mm/s), and the gray scale changes and the time for exposure in target location were recorded. Then the focal point of the HIFU therapeutic system was moved over to the opposite site. Instead of the physiological saline solution, the enhancement agent for HIFU treatment as prepared in Example I-1-3 was administered intravenously, the injection speed and the time being the same as that of the control lobe of liver. Then the right side of the rabbit liver (Group A) or the left side of the rabbit liver (Group B) was exposed to HIFU. The treatment modes used for both sides of the liver of the same rabbit were the same.

The rabbits were sacrificed and dissected at 24 hours after HIFU treatment. The dimensions (length, width and thickness) of the coagulation necrosis zone of the rabbit liver lesions were measured. The volume of coagulation necrosis was calculated according to the formula of V=4/3π× 1/2 length × 1 /2 width × 1/2 thickness. The EEF (energy efficiency factor) was calculated according to the expression

*EEF* = *^{ηPt}*/*_{V},* (J/mm³). The EEFs were compared between and within Group A and Group B. η refers to the focusing coefficient of HIFU transducer, which reflects the ultrasound energy focusing capacity of the transducer, here η=0.7; P refers to the total acoustic power of a HIFU source (W); t refers to the total time of HIFU treatment (s); V refers to the volume of HIFU-induced lesions (mm³). A substance that decreases the EEF of the target tissue after its administration is more suitable to be used as the enhancement agent for HIFU treatment according to the present invention. The results are shown in Table 4.

**Table 4**

| EEF of the control lobe and the experimental lobe | | | | |
|---|---|---|---|---|
| | Group A | Group B | Totaling | P value |
| Control lobe | 7.09 ± 4.11 | 6.67 ± 3.13 | 6.87 ± 3.60 | > 0.5* |
| Experimental lobe | 2.73 ± 1.64 | 3.43 ± 2.07 | 3.10 ± 1.89 | > 0.5* |
| P value | < 0.001 | < 0.001 | < 0.001 | |

The results in Table 4 indicate that there were no notable differences between the rabbits in Group A and those in Group B that were administered with physiological saline solution; also, there were no notable differences between rabbits in Group A and those in Group B that were administered with the particle enhancement agent for HIFU treatment as prepared in Example I-1-3. However, when comparing the experimental results of the control lobe with those of the experimental lobe, there were significant differences in both Group A and Group B. When combining the results of Group A and Group B, it could be seen that the EEF of the experimental lobe greatly decreased. In fact, the EEF of the control lobe which was administered with physiological saline solution is about 2.22 times as much as the EEF of the experimental lobe.

### Animal test 2 Combined use of the particle enhancement agent for HIFU treatment as prepared in Example I-1-1 and HIFU therapeutic devices

Thirty New Zealand white rabbits each weighing approximately 2kg, which were provided by the Laboratory Animals Center of Chongqing University of Medical Sciences, were divided into an experimental group and a control group randomly with 15 rabbits for each group. Two exposure spots were introduced on each rabbit. The rabbits in the control group were administered with physiological saline solution (dosage: 2.5ml/kg) by rapid injection via rabbit ear border vein. The rabbits in the experimental group were administered with emulsified iodized oil as prepared in Example 1-1-1 (dosage: 2.5ml/kg) by rapid injection via rabbit ear border vein followed by flushing with 1ml physiological saline solution in order to ensure that the emulsified iodized oil had entered into the body completely. One hour later, a High-intensity Focused Ultrasound Tumor Therapeutic System Model-JC manufactured by Chongqing Haifu (HIFU) Technology Co. Ltd. was used to radiate the livers of the white rabbits in the experimental group and the control group under single pulse exposure. The power for exposure was 220W; the frequency was 1.0MHZ; the exposure depth was 20mm and the exposure was stopped when coagulative necrosis occurred. The measured data were expressed with mean value ± SD, processed by the statistics software SPSS 10.0 for Windows using independent and paired sample test. The enumeration data were determined by using chi-square (χ²) test. The comparisons between the EEF of the control group and the EEF of the experimental group are shown in Table 5.

**Table 5 Comparisons between the EEFs of the control group and the experimental group**

| Group | N | EEF (χ±s) (J/mm³) |
|---|---|---|
| Control group | 30 | 31.05 ± 2.68 |
| Experimental group | 30 | 7.16 ± 1.38* |

| | | |
|---|---|---|
| N refers to the numbers of the exposure spots. *P<0.001 when compared to the control group. | | |

The results in Table 5 show that the emulsified iodized oil as prepared in Example I-1-1 could greatly reduce the level of EEF for causing lesions of hepatic tissue with HIFU treatment.

### Animal test 3 In vitro study of the particle enhancement agent for HIFU treatment as prepared in Example I-1-3

Ten New Zealand white rabbits (about 3 months old) with no limitation on sex, which were provided by the Laboratory Animals Center of Chongqing University of Medical Sciences, were randomly divided into an experimental group (which was administered with the enhancement agent for HIFU treatment as prepared in Example I-1-3) and a control group (which was administered with physiological saline solution). The rabbits in the two groups weighed 2.40 ± 0.45kg and 2.32 ± 0.08kg (P>0.5), respectively. These rabbits were fasted for 24 hours before experiments. HIFU gynaecological therapeutic apparatus CZF-1, manufactured by Chongqing Haifu (HIFU) Technology Co. Ltd., was used to radiate the rabbit livers. The HIFU gynaecological therapeutic apparatus CZF-1 is composed of a power source, an applicator and circulating water as described in Chinese Patent No. 01144259.X. The parameters in this test were set up as follows: power: 4.05W; frequency: 11MHz; and pulse: 1000Hz.

After the white rabbits were anaesthetized through intramuscular injection, the enhancement agent for HIFU treatment as prepared in Example I-1-3 was delivered via rabbit ear border vein to the rabbits in the experimental group at 50-60 drops/minute for 20 minutes and the physiological saline solution was delivered to those in the control group at 50-60 drops/minute for 20 minutes.

One hour after transfusion, the rabbit was fastened to a workbench in supine position. For each rabbit, the laparotomy was carried out with a 4-5cm incision in the midsection and the rabbit liver in the abdominal cavity was exposed and pulled out slightly after the abdomen wall was opened layer by layer. One or two exposure spots on each liver lobe were introduced for each exposure duration of 3s, 6s, and 9s. The experiments were carried out using the parameters as mentioned above after the exposure spots were introduced. After the lesions were generated, the rabbit liver was put back to the abdominal cavity and the abdomen wall was sutured layer by layer.

In the next day, the rabbits were sacrificed by excessive anaesthetization. The livers were removed and photographed. The dimensions of the lesions were measured and the EEF was calculated. All data were expressed with mean value ± SD, processed by the statistics software SPSS 10.0 for Windows, and used the independent sample test. The statistics was significant when P value was less than 0.05. In this test, 21 exposure spots for each exposure duration of 3s, 6s, and 9s and 63 (21×3) exposure spots in total were obtained in the control group; 30 exposure spots for each exposure duration of 3s, 6s, and 9s and 90 (30 × 3) exposure spots in total were obtained in the experimental group. The EEF was calculated with the above-mentioned expression, and the results are shown in Table 6.

**Table 6**

| The EEF of the control group and the experimental group | | | | |
|---|---|---|---|---|
| | | Exposure duration | | |
| | n | 3s | 6s | 9s |
| Control group | 21 | 0.2749 ± 0.2409 | 0.1783 ± 0.0733 | 0.1846 ± 0.0896 |
| Experimental group | 30 | 0.1177 ± 0.0609 | 0.1367 ± 0.0613 | 0.1463 ± 0.069 |
| P Value | | <0.0 1 | <0.05 | > 0.05 |

The results in Table 6 show that the EEF for each exposure duration of 3s, 6s, and 9s in the control group were 2.34, 1.30 and 1.26 times the EEF for each exposure duration of 3s, 6s, and 9s in the experimental group, respectively. In fact, the mean of the EEF in the control groups (3s, 6s, and 9s) is 1.59 times as much as the mean of the EEF in the experimental groups (3s, 6s, and 9s). If the data obtained in the exposure duration of 9s, which the difference of the EEF between the control group and the experimental group was not statistically significant, were not considered, the mean of the EEF in the control groups (3s, and 6s) is 1.78 times as much as the mean of the EEF in the experimental groups (3s, and 6s).

### Animal test 4 Combined use of the particle enhancement agent for HIFU treatment as prepared in Example I-2-1 and HIFU therapeutic devices

### (1) Study on lesions of New Zealand white rabbit liver

Twenty New Zealand white rabbits weighing 2.21±0.56kg, which were provided by the Laboratory Animals Center of Chongqing University of Medical Sciences, were used. These rabbits were shaved at the lower bosom and the midsection on the day prior to study. A High-intensity Focused Ultrasound Tumor Therapeutic System Model-JC manufactured by Chongqing Haifu (HIFU) Technology Co. Ltd. was used to radiate these white rabbits. The High-intensity Focused Ultrasound Tumor Therapeutic System Model-JC is composed of an adjustable power generator, a B-mode ultrasound monitoring system, a therapeutic transducer, a mechanical motion control system, a treatment bed, and an acoustic coupling device. The therapeutic transducer of the System, with working frequency of 1MHz, diameter of 150mm, and focal distance of 150mm, using standard circulating degassed water with gas content of less than or equal to 3ppm, can produce a focal region of 2.3×2.4×26mm and deliver an average acoustic intensity of 5500W/cm². The transducer used in this study was 150mm in diameter, and it had a focal distance of 135mm, a working frequency of 1.0MHz and an acoustic power of 200W. The exposure depth was 20mm, and a discontinuous single pulse exposure with exposure duration of 3s and interval of 5s was applied. The physiological saline solution (0.02ml/kg) was quickly delivered via rabbit ear border vein to each rabbit, and the rabbit liver was exposed with HIFU using single pulse exposure 60 seconds later for the control side. The enhancement agent for HIFU treatment as prepared in Example I-2-1 (0.02ml/kg) was quickly delivered via rabbit ear border vein to each rabbit, and the other plane of the same rabbit liver of the control side was exposed to HIFU at 60 seconds later for the experimental side. The ultrasound exposures finished when gray-scale changes occurred at the target location. If there are no gray-scale change to be seen, the total exposure duration should be no more than 20s. Three days after ultrasound exposure, the rabbits were sacrificed by breaking their necks and were then dissected. The volume (V) of coagulative necrosis of rabbit liver was measured. The EEF was calculated according to the expression of *EEF* = *^{ηPt}*/*_{V},*
wherein, t refers to the exposure time, η=0.7. The median of the EEFs was 6.0160 on the control side and 1.2505 on the experimental side. Wilcoxon signed rank-sum test showed Z=-2.485, and P=0.013. The results of this study show that the fluoro-carbon emulsion increases the effectiveness of HIFU to cause lesions of the rabbit livers. In fact, the mean of the EEF in the control side is 4.81 times as much as the mean of the EEF in the experimental side.

### (2) Study on lesions of goat liver

Twenty Nanjiang yellow goats weighing 22.25±4.51kg were used. The goats were shaved at the right bosom and the right abdomen on the day of the study. A High-intensity Focused Ultrasound Tumor Therapeutic System Model-JC manufactured by Chongqing Haifu (HIFU) Technology Co. Ltd. was used to radiate these yellow goats. The transducer used in this study was 150mm in diameter, had a focal distance of 135mm, a working frequency of 0.8MHz, and an acoustic power of 220W. The exposure depth was 30mm, and a discontinuous single pulse exposure with exposure duration of 3s and interval of 5s was applied. Ribs of all the goats were not removed. A pre-scan was carried out before HIFU exposure and the areas for exposure including 4 planes were selected. One exposure spot was introduced on each plane, and two-dimensional ultrasound was used to monitor rib clearance. The physiological saline solution (0.02ml/kg) was quickly delivered intravenously via ear border to each goat, and the goat liver was exposed to HIFU 60 seconds later, and two exposure spots were introduced on each goat on the control side. The enhancement agent for HIFU treatment as prepared in Example I-2-1 (0.02ml/kg) was quickly delivered intravenously via ear border to each goat, and the goat liver was exposed to HIFU 60 seconds later, and two exposure spots were introduced on each goat on the experimental side. When gray-scale changes occurred at the target location, the exposures were repeated another 4 or 5 times. If there is no gray-scale change to be seen, the total exposure duration should be no more than 200s. Three days after ultrasound exposure, these goats were sacrificed and dissected. The volume (V) of coagulative necrosis of goat liver was measured. The EEF was calculated according to the expression of *EEF* = *^{ηPt}*/*_{V},*
wherein, T refers to the exposure time, η=0.7. The median of EEFs was infinite for the control side and 5.1904 for the experimental side using the combination of HIFU and fluoro-carbon emulsion. Wilcoxon signed rank-sum test showed P=0.004. This study showed that the efficiency to cause lesions of the goat livers with HIFU improved significantly by using the fluoro-carbon emulsion without removal of the ribs of the goats.

### (3) Study on lesions of goat kidney

Twenty Nanjiang yellow goats weighing 22.25±4.51kg were used. These goats were shaved at the right bosom and the right abdomen on the day of the study. A High-intensity Focused Ultrasound Tumor Therapeutic System Model-JC manufactured by Chongqing Haifu (HIFU) Technology Co. Ltd. was used to radiate these yellow goats. The transducer used in this study was 150mm in diameter, and it had a focal distance of 135mm, a working frequency of 0.8MHz and an acoustic power of 220W. The exposure depth was 20mm, and a discontinuous single pulse exposure with exposure duration of 3s and interval of 5s was applied. Ribs of all the goats were not removed. A pre-scan was carried out before HIFU exposure and the areas for exposure including 1 plane on the upper pole of the kidney and 1 plane on the lower pole of the kidney respectively were selected. One exposure spot was introduced on each plane, and two-dimensional ultrasound was used for observation. The right ribs would be avoided if they become obstacles. The physiological saline solution (0.02ml/kg) was quickly delivered intravenously via ear border to each goat, and the goat kidney was exposed to HIFU under single pulse exposure 30 seconds later on the control side. The enhancement agent for HIFU treatment as prepared in Example I-2-1 (0.02ml/kg) was delivered quickly intravenously via ear border to each goat, and the goat kidney was exposed to HIFU 60 seconds later on the experimental side. When gray-scale changes occurred at the target location, the exposures were repeated another 3 or 4 times. If there is no gray-scale change to be seen, the total exposure duration should be no more than 150s. Three days after ultrasound exposure, these goats were sacrificed and dissected. The volume (V) of coagulative necrosis of goat kidney was measured. The EEF was calculated according to the expression of *EEF* = *^{ηPt}*/*_{V},* wherein, T refers to the exposure time, η=0.7. The EEFs were 10.58±3.95 for the experimental side and 486.37±215.41 for the control side. Wilcoxon signed rank-sum test shows that P=0.008. The results of this study indicate that the fluoro-carbon emulsion greatly increased the ability of HIFU to cause lesions in normal goat kidneys. In fact, the mean of the EEF in the control side is more than 40 times the mean of the EEF in the experimental side.

### Animal test 5 Combined use of the plasmid enhancement agent for HIFU treatment as prepared in Example II-1 and HIFU therapeutic devices

Thirty-six New Zealand white rabbits weighing approximately 2kg, which were provided by the Laboratory Animals Center of Chongqing University of Medical Sciences, were randomly divided into one control group and two experimental groups using the HIFU enhancement agent, 12 rabbits for each group. The rabbits in the control group were administered with physiological saline solution (2ml/kg) by rapid injection via rabbit ear border vein. The rabbits in the experimental groups were administered with the agent as prepared in Example II-1 (2ml/kg) by rapid injection via rabbit ear border vein and then flushed with 1ml physiological saline solution in order to ensure that the agent had entered into the body completely. Two experimental groups were exposed to HIFU at 24 hours and 48 hours after injection of agent, respectively. The group that was exposed to HIFU at 24 hours after injection was called the first experimental group and the group that was exposed to HIFU 48 hours after injection was called the second experimental group. A High-intensity Focused Ultrasound Tumor Therapeutic System Model-JC manufactured by Chongqing Haifu (HIFU) Technology Co. Ltd. was used to radiate the livers of the rabbits in the control group and two experimental groups under single pulse exposure. The High-intensity Focused Ultrasound Tumor Therapeutic System Model-JC is composed of an adjustable power generator, a B-mode ultrasound monitoring system, a therapeutic transducer, a mechanical motion control system, a treatment bed, and an acoustic coupling device. The therapeutic transducer of the System, with working frequency of 1MHz, diameter of 150mm, and focal distance of 150mm, using standard circulating degassed water with gas content of less than or equal to 3ppm, can produce a focal region of 2.3x2.4x26mm and deliver an average acoustic intensity of 5500W/cm². In this test, the acoustic power for exposure was 220W, the frequency was 1.0MHZ, the exposure depth was 20mm, and the exposure duration was 15 seconds. The animals were sacrificed and dissected after HIFU exposure. The dimensions of coagulation necrosis at target location were measured. The EEFs needed to produce certain coagulative necrosis in the rabbit livers in the control group and two experimental groups are shown in Table 7.

**Table 7**

| Group | Number of the Exposure spot | V (mm³) | EEF (J/mm³) |
|---|---|---|---|
| Control group | 24 | 582.50± 353.93 | 7.39± 4.99 |
| First experimental group | 45 | 1281.56± 884.56 | 2.71 ± 1.29 |
| Second experimental group | 17 | 1525.63± 1007.46 | 2.25± 1.61 |

As shown in Table 7, the volumes of coagulative necrosis induced in both of the experimental groups during HIFU treatment under the same conditions with the exposures carried out either at 24 hours or 48 hours after injection for the same exposure duration, were greater than that of the control group, and the EEF needed in the experimental groups decreased greatly in comparison with that of the control group. The differences in volumes of coagulative necrosis and the EEFs between the control group and the experimental groups were statistically significant (P<0.05).

### Animal test 6 In vivo study of the plasmid enhancement agent for HIFU treatment as prepared in Example III

Forty New Zealand white rabbits weighing averagely 2.7±0.3kg with no limitation on sex, which were provided by the Laboratory Animals Center of Chongqing University of Medical Sciences, were randomly divided into three HAP groups and one control group, 10 rabbits for each group.

Twenty-four hours before HIFU treatment, each rabbit in HAP groups was administered with HAP suspensions as prepared in Example III varying in concentrations by rapid injection (< 5s) via rabbit ear border vein with a dosage of 2-3ml per 1kg body weight. Then they were flushed with 1ml physiological saline solution in order to ensure that the suspension had entered into the body completely. Each rabbit in the control group was administered with physiological saline solution (2ml/kg) by rapid injection via rabbit ear border vein. The rabbits were denuded with 8% sodium sulfide on the right bosom and abdomen. The rabbits were anesthetized by an intramuscular injection of Sumianxin (0.2ml/kg), prior to HIFU treatment, and the abdomen wall was incised under aseptic conditions to fully expose the liver.

HIFU gynaecological therapeutic apparatus CZF-1 manufactured by Chongqing Haifu (HIFU) Technology Co. Ltd. was used to radiate the rabbit livers. The HIFU gynaecological therapeutic apparatus CZF-1 is composed of a power source, an applicator, and circulating water as disclosed in Chinese Patent No. 01144259.X. The parameters in this test were set up as follows: frequency: 9.85MHz, power: 5W, focal distance: 4mm, and treatment mode: single pulse exposure. Three exposure spots for one cycle and 2 or 3 exposure cycles for each liver were introduced. The exposure duration was 10 seconds. The incision was sutured after HIFU treatment. Twenty-four hours later, the rabbits were sacrificed by rapid injection of 10ml air via rabbit ear border vein. The dimensions of coagulation necrosis formed at target location were measured and the EEF was calculated.

T-test and relative analysis were used for comparisons between groups.

Dot-shaped gray-white coagulative necrosis appeared in the treated region immediately after HIFU treatment and the boundary between the tissue lesions and the normal tissue was clear. After the rabbits in these groups were exposed to HIFU for the same duration, the volume of focal regions (coagulative necrosis) formed in the HAP groups with different HAP dosages was greater than that in the control group administered with physiological saline solution; the EEF needed in HAP groups decreased greatly in comparison with the control group, and the difference between the HAP groups and the control group were very statistically significant (P<0.001). By comparing the different HAP groups with different nanometer-sized HAP dosages, it is shown that when the dosage of HAP was increased, the volume of focal regions (coagulative necrosis) formed was increased greatly; the EEF needed in HAP groups decreased greatly, which again was very statistically significant (P<0.001). Table 8 shows the volume of focal regions (coagulative necrosis) and the EEFs in the HAP groups with different HAP dosages after HIFU treatment (x̅ ±s).

**Table 8**

| Group | Dosage | n | V/mm³ | EEF |
|---|---|---|---|---|
| Control group | 2ml/kg | 30 | 95.3±21.6 | 0.39±0.09 |
| HAP group 1 | 50mg/kg | 30 | 153.1±41.8 | 0.24±0.05 |
| HAP group 2 | 100mg/kg | 25 | 223.2±55.1 | 0.19±0.01 |
| HAP group 3 | 150mg/kg | 21 | 287.7±47.9 | 0.13±0.00 |

| | | | | |
|---|---|---|---|---|
| Note: "n" in the table refers to the number of the exposure spots. | | | | |

From the study above, it can be concluded that the nanometer-sized HAP can greatly enhance the therapeutic effects of HIFU *in vivo* and that HIFU treatment was more effective when more HAP dosage was applied.

### Animal test 7 In vitro study of the plasmid enhancement agent for HIFU treatment as prepared in Example III

Eighty healthy New Zealand rabbits weighing 2.5±0.3kg with no limitation on sex, which were provided by the Laboratory Animals Center of Chongqing University of Medical Sciences, were fasted for 24 hours before HIFU treatment. Then, each rabbit was administered with HAP milk-white suspension with a concentration of 25g/L as prepared in Example III (2ml/kg) by rapid injection via rabbit ear border vein and flushed with 1ml physiological saline solution. The rabbit livers were scanned with HIFU at 24 hours after administration of HAP (20 rabbits), 48 hours (25 rabbits), 72 hours (10 rabbits) and 168 hours (15 rabbits), respectively. Ten rabbits in the control group were administered with physiological saline solution (2ml/kg) and the rabbit livers were scanned with HIFU at 24 hours after the administration of physiological saline solution. Prior to the HIFU treatment, these rabbits were denuded with 8% sodium sulfide on the right bosom and abdomen. The rabbits were anesthetized by an intramuscular injection of Sumianxin(0.2ml/kg) and secured to a High-intensity Focused Ultrasound Tumor Therapeutic System Model JC-A.

The High-intensity Focused Ultrasound Tumor Therapeutic System Model JC-A was manufactured by the Institute of Ultrasound Engineering in Medicine, Chongqing University of Medical Sciences, and the manufacture thereof was approved by the State Food and Drug Administration in China with the registration No. 99-301032. This system consists of a real time ultrasound monitoring and positioning apparatus and a therapeutic apparatus. Circulating degassed water was used as the acoustic coupling agent, which contained a gas of less than 3×10⁻⁶. Therapeutic parameters were set up as follows: power: 220W, frequency: 1MHz, focal distance: 150mm and focal length: 12mm. The therapeutic applicator could move in the directions of X, Y and Z-axis freely.

The bosom and abdomen of each rabbit was immersed in the circulating degassed water and the rabbit liver was imaged clearly under B-mode ultrasound. One or two exposure spots could be introduced on each liver under single pulse exposure. Each exposure spot was introduced for a fixed exposure period of 15s with an exposure depth of 20mm. Then, the rabbits were sacrificed by rapid injection of 10ml air via rabbit ear border vein at 24 hours after HIFU treatment, and the liver was exteriorized and incised along the acoustic pathway, showing the section of maximum coagulative necrosis area. Then the shape of the coagulative necrosis area was determined, and the dimensions of the coagulative necrosis area as determined by TTC-staining were measured. Then the EEF was calculated.

T-test and relative analysis were used for comparisons between groups.

Under the same treatment conditions, the coagulative necrosis area formed in the HAP groups using nanometer-sized HAP was larger than that in the control group (p<0.05); the EEF needed for HIFU treatment in the HAP groups decreased greatly in comparison with the control group. Also, in the HAP groups, the largest coagulative necrosis area was obtained with HIFU exposure at 24 hours and 48 hours after the HAP injection, and accordingly the least EEF was needed. It is probably most effective to carry out the HIFU exposure at 24 hours and 48 hours after the HAP injection. If the time to carry out the HIFU exposure after the HAP injection were postponed, a smaller necrosis area would be formed. Nevertheless, even at 2 weeks after the HAP injection, it was shown that the HIFU treatment was more effective in comparison with the control group (p<0.05) (see Table 9).

**Table 9**

| Comparisons between HAP groups performing HIFU exposure in different intervals after the HAP injection and the control group | | | | |
|---|---|---|---|---|
| Intervals after HAP injection | n | Average exposure period (s) | Average volume of focal region (mm³) | Average EEF |
| Control group | 16 | 15 | 546.67 | 7.39± 4.99 |
| 24 hours | 57 | 15.88 | 1291.56 | 2.68 ± 1.29 |
| 48 hours | 17 | 15.6 | 1525.63 | 2.32 ± 1.61 |
| 72 hours | 27 | 16.15 | 1153.26 | 3.28± 1.35 |
| 168 hours | 26 | 15 | 920 | 2.51± 0.87 |

| | | | | |
|---|---|---|---|---|
| Note: "n" in the table refers to the actual numbers of the exposure spots. | | | | |

From the experiments as above, it can be found that the nanometer-sized HAP can greatly enhance the therapeutic effects of HIFU *in vitro,* and the HIFU treatment was more effective when the HIFU exposures were carried out at 48 to 72 hours after the HAP injection.

### Animal test 8 Combined use of the microbubble enhancement agent for HIFU treatment and HIFU therapeutic devices

Forty New Zealand white rabbits weighing approximately 2kg were divided into a control group and an experimental group randomly, 20 rabbits for each group. The control group was injected with physiological saline solution (0.05ml/kg). The experimental group was injected with Quanfuxian microbubble ultrasound contrast agent purchased from Nan Fang Hospital (0.05ml/kg) by rapid injection via rabbit ear border vein and then flushed with 1 ml physiological saline solution in order to ensure that the agent had entered into the body completely. One minute later, a High-intensity Focused Ultrasound Tumor Therapeutic System Model-JC manufactured by Chongqing Haifu (HIFU) Technology Co. Ltd. was used to radiate the livers of these rabbits. The exposures were carried out under the power of 200W at frequency of 1.0MHz with exposure depth of 20mm for a certain exposure period. Three days later, the dimensions of lesions induced in rabbit livers were measured and the EEFs calculated. The measured data were expressed with mean value ±SD, processed by the statistics software SPSS 10.0 for Windows using independent and paired sample test. The enumeration data was determined by using chi-square (χ²) test. The results are shown in the following Table 10.

**Table 10**

| The EEFs of the control group and the experimental group | |
|---|---|
| Group | EEF(J/mm³) |
| Control group | 12.83 ± 10.99 |
| Experimental group | 2.70 ± 1.29* |

| | |
|---|---|
| * P < 0.001 when compared to the control group. | |

The results in Table 10 show that the microbubble enhancement agent for HIFU treatment could greatly reduce the level of EEF for causing lesions of hepatic tissue with HIFU treatment.

### INDUSTRIAL APPLICABILITY

The enhancement agent for High-Intensity Focused Ultrasound (HIFU) treatment of the present invention can change the acoustic environment of the target location greatly and can reduce the acoustic energy needed to cause lesions of a target tissue (tumor and non-tumor tissue) per unit volume of the tissue during HIFU treatment. Accordingly, deep-seated and large-sized tumors can be treated with HIFU treatment more effectively under a certain acoustic power without damaging the normal tissues along the acoustic pathway. It becomes possible to use the enhancement agent for HIFU treatment of the present invention for applying HIFU treatment effectively to a patient with a hepatic tumor that is blocked by the ribs in therapeutic acoustic pathway without removal of the ribs.

Although the present invention has been described in connection with the preferred embodiments, it is not intended to limit the scope of the present invention by the above descriptions of the embodiments. It should be understood that various modifications and changes to which the present invention may be applicable will be readily apparent to those skilled in the art. The claims are intended to cover the scope of the present invention.

## Claims

1. An enhancement agent for high intensity focused ultrasound (HIFU) treatment, wherein, the enhancement agent is a substance that is administered to a patient before application of HIFU treatment and can reduce the level of EEF at a target location to be treated with HIFU, the *EEF* = *^{ηPt}*/*_{V},* in unit of J/mm³, refers to HIFU energy needed to effectively treat a tumor per unit volume of the tumor, wherein, η=0.7; P refers to the total acoustic power of a HIFU source (unit: W); t refers to the total time of HIFU treatment (unit: s); V refers to the volume of HIFU-induced lesions (unit: mm³); and
wherein the EEF at the target location before administration of the enhancement agent is defined as EEF_{(base)}, the EEF at the target location after administration of the enhancement agent is defined as EEF₍ₘₑₐₛᵤᵣₑₘₑₙₜ₎, and the ratio between EEF(_{base}) and EEF₍ₘₑₐₛᵤᵣₑₘₑₙₜ₎ is more than 1.

2. The enhancement agent according to claim 1, wherein the ratio between EEF_{(base)} and EEF ₍ₘₑₐₛᵤᵣₑₘₑₙₜ₎ is more than 2.

3. The enhancement agent according to claim 2, wherein the ratio between EEF_{(base)} and EEF₍ₘₑₐₛᵤᵣₑₘₑₙₜ₎ is more than 4.

4. The enhancement agent according to any one of claims 1-3, wherein the enhancement agent can be used for intravenous injection, arterial injection and topical injection and has a particle size ranging from 10nm-8µm.

5. The enhancement agent according to claim 4, wherein the enhancement agent consists of a discontinuous phase which consists of a core encapsulated by a membrane-forming material, and a continuous phase which consists of aqueous medium, the discontinuous phase is uniformly dispersed in the continuous phase, the discontinuous phase has a particle size ranging from 10nm-8µm, the membrane-forming material is biocompatible, and the core is comprised of gas, liquid or nanometer-sized biocompatible solid.

6. The enhancement agent according to claim 5, wherein the membrane-forming material is one or more substances selected from the group consisting of proteins, saccharides or lipids.

7. The enhancement agent according to claim 6, wherein the lipid comprises phospholipin selected from the group consisting of 3-sn-phosphatidylcholine, 1,2-dipalmitoyl-sn-glycero-3-phosphatidylglycerol sodium salt, 1,2-distearoyl-sn-glycero-3-phosphatidylcholine, sodium 1,2-dipalmitoyl-sn-glycero-3-phosphatidate, 1,2-dipalmitoyl-sn-glycero-3-phosphatidylcholine, phosphatidylserine and hydrogenated phosphatidylserine.

8. The enhancement agent according to claim 5, wherein the gas is selected from the group consisting of the air, nitrogen, carbon dioxide, fluorohydrocarbon gas and alkane gas.

9. The enhancement agent according to claim 8, wherein the enhancement agent is an ultrasound contrast agent.

10. The enhancement agent according to claim 5, wherein the liquid is selected from the group consisting of C₅-C₆ alkanes, C₅-C₁₂ fluorohydrocarbons, saturated fatty acid, unsaturated fatty acid and iodized oil.

11. The enhancement agent according to claim 10, wherein the enhancement agent is a fat emulsion for intravenous injection.

12. The enhancement agent according to claim 10, wherein the enhancement agent is an emulsified iodized oil for intravenous injection.

13. The enhancement agent according to claim 10, wherein the enhancement agent is a C₅-C₁₂ perfluorohydrocarbon emulsion for intravenous injection.

14. The enhancement agent according to claim 5, wherein the solid is selected from the group consisting of magnetic biomaterials, hydroxylapatite and calcium carbonate, and the solid has a particle size ranging from 1nm-500nm.

15. The enhancement agent according to claim 14, wherein the solid is hydroxylapatite with a particle size ranging from 1nm-200nm.

16. The enhancement agent according to any one of claims 1-15, wherein the target location is an organ, at which the enhancement agent can arrive via blood circulation.

17. A method for increasing acoustic energy deposition at a target location during HIFU treatment, wherein, the method comprises:
administering the enhancement agent according to any one of claims 1-15 in an effective dosage intravenously via continuous and rapid IV instillation or bolus injection to a patient at 0-168h before the application of HIFU treatment to the target location of a patient.

18. A method for screening an enhancement agent for HIFU treatment, the method comprising:
(a) applying high intensity focused ultrasound (HIFU) to a given tissue, and then calculating the EEF in unit J/mm³ of the tissue according to the expression of *EEF* = *^{ηPt}*/*_{V},* to obtain EEF(_{base}), wherein, η=0.7; P refers to the total acoustic power of a HIFU source (unit: W); t refers to the total time of HIFU treatment (unit: s); V refers to the volume of HIFU-induced lesions (unit: mm³);
(b) administering a candidate enhancement agent to the biological tissue;
(c) calculating the EEF of the tissue after the administration of the enhancement agent to obtain EEF₍ₘₑₐₛᵤᵣₑₘₑₙₜ₎; and
(d) comparing the EEF ₍ₘₑₐₛᵤᵣₑₘₑₙₜ₎ with the EEF_{(base)} of the tissue and selecting a candidate enhancement agent that has an EEF₍ₘₑₐₛᵤᵣₑₘₑₙₜ₎ to EEF_{(base)} ratio of more than 1.

19. The method according to claim 18, comprising comparing the EEF₍ₘₑₐₛᵤᵣₑₘₑₙₜ₎ with the EEF_{(base)} of the tissue and selecting a candidate enhancement agent that has an EEF₍ₘₑₐₛᵤᵣₑₘₑₙₜ₎ to EEF_{(base)} ratio of more than 2.

20. The method according to claim 18, comprising comparing the EEF ₍ₘₑₐₛᵤᵣₑₘₑₙₜ₎ with the EEF_{(base)} of the tissue and selecting a candidate enhancement agent that has an EEF₍ₘₑₐₛᵤᵣₑₘₑₙₜ₎ to EEF_{(base)} ratio of more than 4.
